# EUROPÄISCHE PATENTSCHRIFT

(11) **EP 3 419 677 B1**
(45) Veröffentlichungstag und Bekanntmachung des Hinweises auf die Patenterteilung: **08.04.2020**
(21) Anmeldenummer: 16836222.6
(22) Anmeldetag: 28.12.2016
(51) Int. Cl.: A61L 27/06, A61F 2/38, A61L 27/10, A61L 27/18, A61L 27/30

(54) **ORTHOPÄDISCHES IMPLANTAT**
ORTHOPEDIC IMPLANT
IMPLANT ORTHOPÉDIQUE

(30) Priorität: 29.12.2015 DE 102015016895
(43) Veröffentlichungstag der Anmeldung: 02.01.2019
(73) Patentinhaber: ZM Präzisionsdentaltechnik GmbH, 18055 Rostock (DE)
(72) Erfinder: MITROVIC, Milija, 1805 Rostock (DE)
(86) Internationale Anmeldenummer: PCT/DE2016/000441
(87) Internationale Veröffentlichungsnummer: WO 2017/114519

(56) Entgegenhaltungen:
- EP-A1- 2 742 905
- WO-A1-2012/126449
- DE-A1- 19 711 628
- ZOTHNER A ET AL: "Die Evolution des Abutments", QUINTESSENZ DER ZAHNTECHNIK, QUINTESSENZ VERLAGS, DE , Bd. 35, Nr. 5 1. Januar 2009 (2009-01-01), Seiten 2-16, XP002676248, ISSN: 0340-4641 Gefunden im Internet: URL:http://www.memodent.nl/upload/content/ documents/qz_05_2009_zothner.pdf

## Beschreibung

Die Erfindung betrifft ein orthopädisches Implantat in Form einer Knieendoprothese mit einer femoralen Komponente aus einem metallischen Werkstoff, bei dem die Gleitflächen der femoralen Komponente als Einsätze ausgebildet sind, die in den metallischen Grundkörper der femoraten Komponente eingesetzt sind und aus einem keramischen Werkstoff auf der Basis von Zirkoniumdioxid, Aluminiumoxid bzw. einer Mischung aus beiden bestehen, und bei dem die Verbindung zwischen den Einsätzen und dem metallischen Grundkörper der femoralen Komponente über ein silikatisches Glaslot hergestellt ist.

Beim künstlichen Ersatz eines Kniegelenkes werden die beeinträchtigten Gelenkflächen entfernt und durch zwei voneinander unabhängige Implantate auf der tibialen und femoralen Seite, d.h. am oberen Ende des Schienbeins (Tibia) und am unteren Ende des Oberschenkelknochens (Femur), ersetzt. Als Gelenkflächenersatz auf der femoralen Seite dient dabei ein Implantat, das bei den meisten bekannten Implantaten entweder aus einer speziellen hochfesten Titanlegierung oder aus einer aus Kobalt-Chrom-Legierung besteht, die gegossen oder geschmiedet und anschließend poliert wird. Dabei wird die tibiale Gelenkseite durch ein aus der Titan- oder Kobalt-Chromlegierung bestehendem Implantat ersetzt und es wird auf diesem in der Regel ein Polyethyleneinsatz befestigt, der als Gleitpartner für die femorale Komponente dient.

Der künstliche Kniegelenkersatz ist heute eine zuverlässige Operation mit Standzeiten der Implantate von bis zu 15 Jahren. Die häufigste Versagensursache ist die aseptische Implantat-Lockerung, die durch Abriebpartikel der tibialen Polyethylenkomponente hervorgerufen wird und die die Lebensdauer eines Implantats erheblich verkürzen kann. Die Abriebpartikel entstehen durch den Verschleiß der Polyethylenkomponente, hervorgerufen durch die Reibung mit der femoralen Komponente. Wenn die Abriebpartikel in das Knochen-Implantat-Interface gelangen, werden biologische Prozesse an den Grenzflächen Implantat-Knochen bei zementfreier Verankerung oder Implantat-Knochenzement-Knochen bei zementierter Verankerung ausgelöst, die zu einer progressiven lokalen Knochendestruktion und schließlich zur Lockerung der Implantate führen können. Bei einem vollständigen Aufbrauch der Polyethylenkomponente der Tibia kann es in der Folge außerdem zu einem Verschleiß des darunter befindlichen metallischen Tibiaplateaus und zu allergischen Reaktionen mit den dann freigesetzten Metallionen kommen.

Neben den metallischen Knieendoprothesen wird unter dem Handelsnamen Oxinium® eine Zirkonium-Niob-Legierung eingesetzt, deren femorale Komponente in einem Wärmebehandlungsprozeß bei etwa 500 °C in eine Zirkoniumoxidkeramik umgewandelt wird, die gleichsam eine Beschichtung aus Zirkoniumdioxid darstellt. Femer sind Implantate bekannt geworden, bei denen eine femorale Komponente aus einer Mischkeramik (BioloxDelta®) eingesetzt wird. Der Verschleiß des Polyethylens wird durch die Verwendung dieser auf dem Markt erhältlichen femoralen Komponente im Vergleich zu Metall/Polyethylen-Gleitpaarungen verringert und auf diese Weise die Haltbarkeit der Knieendoprothese verlängert. Als Nachteile dieser bekannten Komponenten sind jedoch die hohen Fertigungskosten der Oxinium-Komponenten sowie die Sprödigkeit und die mangelnde Haftfestigkeit der keramischen Komponente am Knochenzement bzw. am Knochen zu sehen.

Neben diesen bekannten Implantaten ist auch aus er DE 10 2011 015 300 A1 und der WO 2012/126449 ein Implantat der eingangs genannten Art in Form einer Knieendoprothese aus einem metallischen Werkstoff bekannt geworden, bei dem die Gleitflächen der femoralen Komponente bereits aus einem keramischen Werkstoff bestehen und bei dem die Verbindung zwischen den Einsätzen und dem metallischen Grundkörper der femoralen Komponente über ein silikatisches Glaslot hergestellt wird.

Weiterhin ist auf dem Gebiet von Zahnimplantaten nach Zothner A. et al "Die Evolation des Abutments" Zeitschrift "Quintessens der Zahntechnik" Bd 35 Nr. 5, Januar 2009, Seiten 2 - 16 eine ähnliche Verbindungstechnik gemäss dem Gattungsbegriff bekannt geworden. !

Aufgabe der Erfindung ist es, ein solches Implantat derart auszubilden, dass die Reibung und damit der Verschleiß zwischen der femoralen Komponente und dem Gleitpartner Polyethylen im Vergleich zu den üblicherweise verwendeten Gleitpaarungen möglichst gering ist und zugleich die Haftfestigkeit der femoralen Komponente im Femurknochen erhöht wird.

Die Erfindung löst diese Aufgabe dadurch, dass die Verbindung zwischen den Einsätzen (3) und dem metallischen Grundkörper durch eine Vorbehandlung des metallischen Grundkörpers über,ein in einem keramischen Brand verfestigtes silikatisches Glaslot erfolgt sowie eine Festlegung üben ein höher als das Glaslot, jedoch unterhalb der Schmelztemperatur des metallischen Grundkörpers schmelzendes Glaslot durchgeführt, wobei im metallischen Grundkörper Lotabzugskanäle vorgesehen sind.

Für eine optimale Osseointegration des Implantates ist es weiterhin von Vorteil, wenn der mit dem Oberschenkelknochen zu verbindende, aus einem metallischen Werkstoff bestehende Grundkörper der femoralen Komponente vorzugsweise aus einer hochfesten Titanlegierung mit einem Wärmeausdehnungskoeffizienten (WAK-Wert) von etwa 10,5 besteht.

Nachfolgend soll die Erfindung anhand eines in der Zeichnung dargestellten Ausführungsbeispiels näher erläutert werden. Es zeigen:
- Fig. 1: eine Knieendoprothese gemäß dem Stand der Technik in perspektivischer Darstellung,
- Fig. 2: eine schematische Darstellung eines an einem Oberschenkelknochen fixierten femoralen Kniegelenkimplantates gemäß der Erfindung,
- Fig. 3: eine schematische Darstellung einer Anordnung aus Grundkörper und Einsatz für die femorale Komponente einer Knieendoprothese gemäß Fig. 2 und
- Fig. 4: eine schematische Darstellung eines vergrößerten Teilbereiches eines Einsatzes gemäß Fig. 3 nach längerer Lebensdauer einer Knieendoprothese.

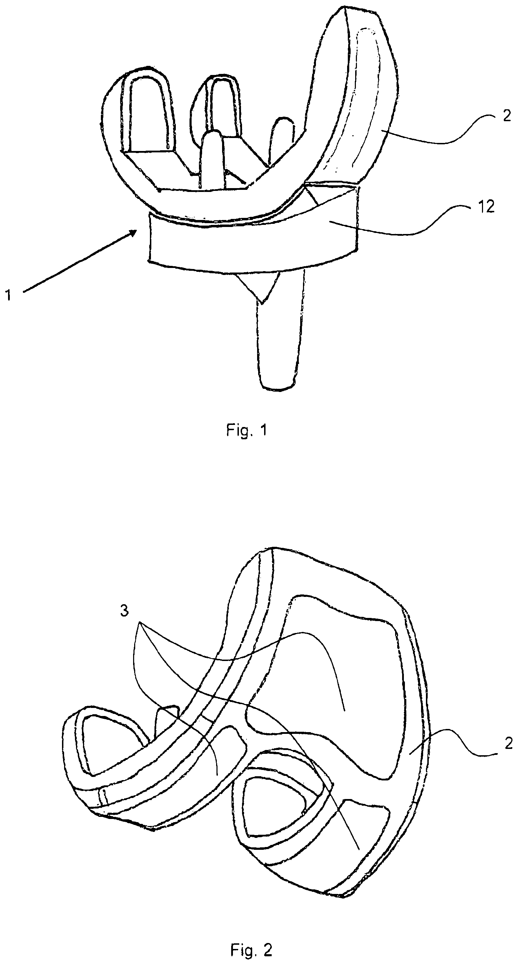

Die Abbildung in Fig. 1 zeigt eine Knieendoprothese 1 gemäß dem Stand der Technik in perspektivischer Darstellung. Das Implantat setzt sich zusammen aus einer Femurkomponente mit einem Grundkörper 2, der aus einem Keramikwerkstoff besteht, sowie einer Tibiakomponente 12, bei der zumindest der der Femurkomponente zugewandte Bereich aus Polyethylen besteht.

Bild 2 zeigt demgegenüber eine Femurkomponente mit in den Grundkörper 2 eingesetzten Gleitflächen, die von Einsätzen 3 aus einem Keramikwerkstoff gebildet werden. Zur Herstellung dieser Einsätze 3 wurde im Fall des hier beschriebenen Ausführungsbeispiels Zirkoniumdioxid verwendet, in gleicher Weise können die Einsätze 3 aber auch aus einer Aluminiumoxidkeramik oder aus einer Mischung dieser beiden Keramiken bestehen.

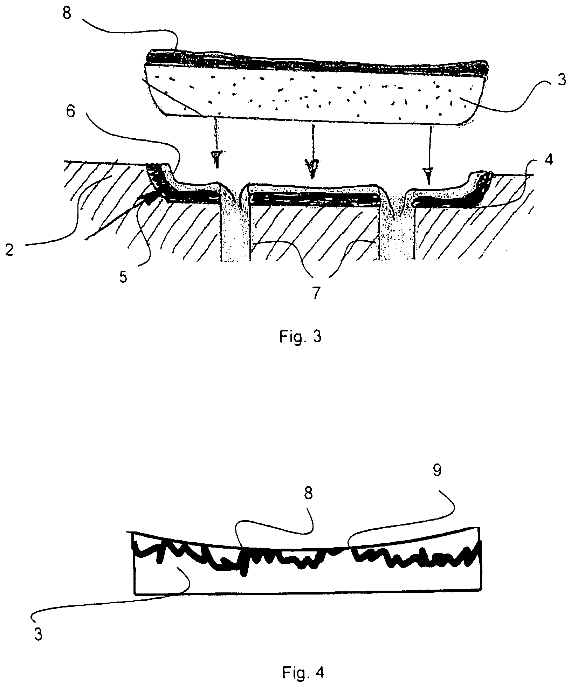

Die keramischen Einsätze 3 sind nach Art von Intarsien derart in zugeordnete "wannenförmige" Aufnahmen 4 eines metallischen Grundkörpers 2 aus einem Titanwerkstoff, im Fall der hier gezeigten Darstellung der hochfesten Titan-Legierung Ti-6Al-4V (Titan Grade 5), der femoralen Komponente 1 integriert, dass sie stufenlos in den Grundkörper 2 übergehen und dass keine Absatzkanten entstehen. Vor dem Hintergrund, dass Titan an sich eine raue Oberfläche hat und dazu beim Erhitzen eine Oxidschicht bildet, die eine homogene Lötung verhindert, muss das Titan durch ein spezielles Brennverfahren zunächst vorbehandelt werden. Die keramischen Einsätze 3 werden dabei durch ein silikatisches Glaslot festgelegt, das in einem keramischen Brand verfestigt und auf diese Weise diese keramischen Einsätze 3 fest mit dem metallischen Grundkörper 2 verbindet. Dies ist durch drei Pfeile an dem in Fig. 3 gezeigten Einsatz 3 angedeutet.

Diese Vorbehandlung ist unbedingt notwendig, da die Größe der zu lötenden Objekte und die Hitzebehandlung einen negativen Einfluss auf die Lötverbindung, wie beispielsweise Brüche in der Titanoxidschicht, bewirken würde. Die Vorbehandlung erfolgt in der Regel mittels eines Airbrush-Verfahrens, bei dem die Schicht aus dem silikatischen Glaslot gleichmäßig aufgesprüht und dem anschließenden Brennprozess unterzogen wird. Durch den Brennprozess entsteht eine Lotschicht 5, die sowohl Titanoxide bindet als auch die Rauigkeit des Titans ausgleicht und somit die optimale Basis für den nachfolgenden Lötprozess bietet.

Um auf dieser Lotschicht 5 eine homogene Glaslotschicht 6 zu erzeugen, bedarf es einer richtigen Schichtstärke zwischen in diesem Fall 0,1 und 0,3 Millimetem sowie dem Vorhandensein von geeigneten Abzugskanälen 7, die zusammen die Voraussetzung für einen homogenen Fluss des Glaslotes 6 in diesem Bereich schaffen. Die in Fig. 3 dargestellten Abzugskanäle 7 im Titan bewirken dabei einen Kapillareffekt, der überschüssiges Glaslot 6 homogen abfließen lässt und das Entstehen von Brüchen in der Titanoxidschicht zuverlässig verhindert.

Nach erfolgter Implantation bilden die Einsätze 3 aus Keramik die äußere Form konventioneller metallischer Femurkomponenten in der Gleitfläche nach und vermindern signifikant den Abrieb zwischen der femoralen Komponente und dem Polyethylen der Tibiakomponente 12. Es ist dieser reduzierte Abrieb der Gleitpartner, der das Risiko der partikelinduzierten aseptische Lockerung mindert und zu längeren Standzeiten der Implantate führt. Indem auch auf den dem metallischen Grundkörper 2 abgewandten Oberseiten der Gleitflächen 3 eine Beschichtung aus einem in diesem Fall weicheren Glaslot 8 vorgesehen ist, wird der Abrieb weiterhin vermindert und die Standzeit des Implantates weiter erhöht. Als Abzugskanäle 7 dienende Bohrungen und eine homogene Verteilung der Vorbeschichtung 5 sind hier nicht erforderlich, da das Glaslot 8 in der Keramik aufgefangen wird bzw. aus dieser herausfließt.

Da die Glaslotbeschichtung 8 geringfügig weicher und damit nachgiebiger gestaltet ist als das keramische Material des Einsatzes 3 in der Gleitfläche, wird, wie abschließend in Fig. 4 schematisch dargestellt ist, im Verlauf der Lebensdauer der Knieendoprothese 1 zusätzlich auch ein kleiner Abtrag aus der Beschichtung 8 der Einsätze 3 in der Gleitfläche der Femurkomponente möglich. Während jedoch in diesem Bereich bei vielen herkömmlichen Konstruktionen ein Abrieb der Metallionen beobachtet werden kann, tritt an dessen Stelle im Falle der vorliegenden Erfindung lediglich ein vergleichsweise harmloser Abrieb 9 der außen liegenden Beschichtung 8 der Einsätze 3 des Keramikmaterials der Femurkomponente aus einem weicheren Glaslot 8, der im wesentlichen Silizium enthält.

## Patentansprüche

1. Orthopädisches Implantat in Form einer Knieendoprothese mit einer femoralen Komponente aus einem metallischen Werkstoff, bei dem die Gleitflächen der femoralen Komponente als Einsätze ausgebildet sind, die in den metallischen Grundkörper der femoralen Komponente eingesetzt sind und aus einem keramischen Werkstoff auf der Basis von Zirkoniumdioxid, Aluminiumoxid bzw. einer Mischung aus beiden bestehen, und bei dem die Verbindung zwischen den Einsätzen und dem metallischen Grundkörper der femoralen Komponente über ein silikatisches Glaslot hergestellt ist, **dadurch gekennzeichnet, dass** die Verbindung zwischen den Einsätzen (3) und dem metallischen Grundkörper (2) durch eine Vorbehandlung des metallischen Grundkörpers (2) über ein in einem keramischen Brand verfestigtes silikatisches Glaslot (5) erfolgt sowie eine Festlegung über ein höher als das Glaslot (5), jedoch unterhalb der Schmelztemperatur des metallischen Grundkörpers (2) schmelzendes Glaslot (6) durchgeführt wird, wobei im metallischen Grundkörper (2) Lotabzugskanäle (7) vorgesehen sind.

2. Implantat nach Anspruch 1, **dadurch gekennzeichnet, dass** die Gleitflächen (3) aus einer Keramik auf der Basis von Zirkoniumdioxid bestehen.

3. Implantat nach Anspruch 2, **dadurch gekennzeichnet, dass** die Gleitflächen (3) aus Zirkoniumdioxid bestehen.

4. Implantat nach Anspruch 1, **dadurch gekennzeichnet, dass** die Gleitflächen (3) aus einer Keramik auf der Basis von Aluminiumoxid bestehen.

5. Implantat nach Anspruch 4, **dadurch gekennzeichnet, dass** die Gleitflächen (3) aus Aluminiumoxid bestehen.

6. Implantat nach einem der Ansprüche 1 bis 5, **dadurch gekennzeichnet, dass** die Gleitflächen (3) stufenlos in zugeordnete wannenförmige Aufnahmen (4) des metallischen Grundkörpers (2) der femoralen Komponente (1) übergehen.

7. Implantat nach einem der Ansprüche 1 bis 6, **dadurch gekennzeichnet, dass** der Grundkörper (2) der femoralen Komponente (1) aus Reintitan besteht.

8. Implantat nach einem der Ansprüche 1 bis 6, **dadurch gekennzeichnet, dass** der Grundkörper (2) der femoralen Komponente (1) aus einer Titan-Legierung besteht.

9. Implantat nach einem der vorangehenden Ansprüche, **dadurch gekennzeichnet, dass** auf den dem metallischen Grundkörper (2) abgewandten Oberseiten der Gleitflächen als Einsätze (3) eine Beschichtung aus einem weicheren Glaslot (8) als das Keramikmaterial der Einsätze (3) vorgesehen ist.

## Claims

1. An orthopaedic implant in the form of a knee endoprosthesis with a femoral component made of a metallic material, in which the sliding surfaces of the femoral component are formed as inserts that are inserted into the metallic main body of the femoral component and consist of a ceramic material based on zirconium dioxide, aluminium oxide or a mixture of the two, and in which the connection between the inserts and the metallic main body of the femoral component is produced via a silicate glass solder, **characterised in that** the connection between the inserts (3) and the metallic main body (2) is established by way of a pretreatment of the metallic main body (2) via a silicate glass solder (5) that is solidified in a ceramic firing, and a fixing is carried out with a glass solder (6) that melts at a temperature higher than the first glass solder (5) yet below the melting point of the metallic main body (2), with solder discharge channels (7) being provided in the metallic main body (2) .

2. The implant according to claim 1, **characterised in that** the sliding surfaces (3) consist of a ceramic based on zirconium dioxide.

3. The implant according to claim 2, **characterised in that** the sliding surfaces (3) consist of zirconium dioxide.

4. The implant according to claim 1, **characterised in that** the sliding surfaces (3) consist of a ceramic based on aluminium oxide.

5. The implant according to claim 4, **characterised in that** the sliding surfaces (3) consist of aluminium oxide.

6. The implant according to any one of claims 1 to 5, **characterised in that** the sliding surfaces (3) transition in a stepless manner into associated trough-shaped receptacles (4) in the metallic main body (2) of the femoral component (1).

7. The implant according to any one of claims 1 to 6, **characterised in that** the main body (2) of the femoral component (1) consists of pure titanium.

8. The implant according to any one of claims 1 to 6, **characterised in that** the main body (2) of the femoral component (1) consists of a titanium alloy.

9. The implant according to any one of the preceding claims, **characterised in that** on the upper sides of the sliding surfaces formed as inserts (3), which upper sides face away from the metallic main body (2), there is provided a coating made of a glass solder (8) that is softer than the ceramic material of the inserts (3).

## Revendications

1. Implant orthopédique sous forme d'une endoprothèse du genou comprenant une composante fémorale en un matériau métallique, dans lequel les faces de coulissement de la composante fémorale sont conçues en tant qu'inserts qui sont insérés dans le corps de base métallique de la composante fémorale et composés d'un matériau céramique à base de dioxyde de zirconium, d'oxyde d'aluminium, respectivement d'un mélange des deux, et dans lequel la liaison entre les inserts et le corps de base métallique de la composante fémorale est réalisée par une brasure au verre silicatique, **caractérisé en ce que** la liaison entre les inserts (3) et le corps de base métallique (2) est effectuée par un prétraitement du corps de base métallique (2) avec une brasure au verre (5) silicatique solidifiée par une cuisson céramique ainsi qu'une fixation par une brasure au verre (6) supérieure à la brasure au verre (5) mais fondant en-dessous de la température de fusion du corps de base métallique (2), dans lequel des canaux de retrait de brasure (7) sont prévus dans le corps de base (2) métallique.

2. Implant selon la revendication 1, **caractérisé en ce que** les surfaces coulissantes (3) sont composées d'une céramique à base de dioxyde de zirconium.

3. Implant selon la revendication 2, **caractérisé en ce que** les surfaces coulissantes (3) sont composées de dioxyde de zirconium.

4. Implant selon la revendication 1, **caractérisé en ce que** les surfaces coulissantes (3) sont composées d'une céramique à base d'oxyde d'aluminium.

5. Implant selon la revendication 4, **caractérisé en ce que** les surfaces coulissantes (3) sont en oxyde d'aluminium.

6. Implant selon l'une des revendications 1 à 5, **caractérisé en ce que** les surfaces coulissantes (3) passent en continu dans des réceptions (4) en forme de cuvette correspondantes du corps de base (2) métallique de la composante fémorale (1).

7. Implant selon l'une des revendications 1 à 6, **caractérisé en ce que** le corps de base (2) de la composante fémorale (1) est composé de titane pur.

8. Implant selon l'une des revendications 1 à 6, **caractérisé en ce que** le corps de base (2) de la composante fémorale (1) est composé d'un alliage de titane.

9. Implant selon l'une des revendications précédentes, **caractérisé en ce qu'**un revêtement dans une brasure de verre (8) plus souple que le matériau céramique des inserts (3) est prévu sur les faces supérieures des surfaces coulissantes en tant qu'inserts (3), détournées du corps de base (2) métallique.
